Europäisches Patentamt

European Patent Office

Office européen des brevets

(19)

(11) Numéro de publication: **0 251 904**
**B1**

# FASCICULE DE BREVET EUROPEEN

(45) Date de publication du fascicule du brevet:
**10.01.90**

(21) Numéro de dépôt: **87401463.2**

(22) Date de dépôt: **25.06.87**

(51) Int. Cl.⁴: **C07D 279/28**

(54) **Procédé de préparation du maléate acide de lévomépromazine.**

(30) Priorité: **26.06.86 FR 8609261**

(43) Date de publication de la demande:
**07.01.88 Bulletin 88/1**

(45) Mention de la délivrance du brevet:
**10.01.90 Bulletin 90/2**

(84) Etats contractants désignés:
**AT BE CH DE ES FR GB GR IT LI LU NL SE**

(56) Documents cités:
**CHEMICAL ABSTRACTS,**
**vol. 63, no. 10, 8 novembre 1965, col. 13279b, Columbus,**
**Ohio, US; & HU-A-152 208 (ELEMER**
**FOGASSY) 22-07-1965**
**CHEMICAL ABSTRACTS,**
**vol. 80, no. 1, 7 janvier 1974, page 312, résumé no. 3541q,**
**Columbus, Ohio, US; & PL-A-66 636 (INSTYTUT**
**FARMACEUTYCZNY) 15-03-1973**
**CHEMICAL ABSTRACTS,**
**vol. 102, no. 23, 10 juin 1985, page 601, résumé**
**no. 203977w, Columbus, Ohio, US; & HU-A-32 813 (EGYT**
**GYOGYSZERVEGYESZETI GYAR) 28-09-1984**

(73) Titulaire: **RHONE-POULENC SANTE, 20, avenue**
**Raymond Aron, F-92160 Antony(FR)**

(72) Inventeur: **Berger, Christian, 26 Chemin de la Forestière**
**Tour No. 3, F-69130 Ecully(FR)**

(74) Mandataire: **Pilard, Jacques et al, RHONE-POULENC**
**INTERSERVICES Service Brevets Pharma 25, Quai Paul**
**Doumer, F-92408 Courbevoie Cédex(FR)**

## Description

La présente invention concerne un nouveau procédé de préparation du maléate acide de lévomépromazine par dédoublement de la (±)(diméthylamino-3 méthyl-2 propyl)-10 méthoxy-2 phénothiazine au moyen de l'acide (-) L-di.p.toluyltartrique suivi de l'action de l'acide maléique.

La lévomépromazine ou (-)(diméthylamino-3 méthyl-2 propyl)-10 méthoxy-2 phénothiazine est un agent thérapeutique particulièrement intéressant comme tranquillisant, neuroleptique et analgésique.

Il est connu de préparer la lévomépromazine par dédoublement de la(±)(diméthylamino-3 méthyl-2 propyl)-10 méthoxy-2 phénothiazine au moyen de divers agents de dédoublement tels que l'acide tartrique (brevet hongrois HU 32 813, Chem. Abstr. 102 203977 w ; brevet hongrois HU 157 158 ; Chem. Abstr. 72 132762 w), l'acide dibenzoyl (d) tartrique (brevet hongrois HU 152 208, Chem. Abstr. 63 13279 b) ou par l'acide O-diacétyl (-) tartrique monoamide (brevet polonais POL 66 636, Chem. Abstr. 80 3541 q). Cependant ces procédés ne conduisent pas toujours à des rendements satisfaisants ou bien nécessitent l'emploi de quantité importante d'agent de dédoublement qui, parfois, peut être d'un accès difficile.

Il a maintenant été trouvé, et c'est ce qui fait l'objet de la présente invention que l'on peut obtenir le maléate acide de lévomépromazine avec de bons rendements à partir de la (±)(diméthylamino-3 méthyl-2 propyl)-10 méthoxy-2 phénothiazine (base racémique) par cristallisation du sel neutre de la base dextrogyre avec l'acide di.p.toluyl-tartrique puis cristallisation directe du maléate acide de la base lévogyre dans les eaux-mères de cristallisation du di.p.toluyl-tartrate de la base dextrogyre.

Selon la présente invention, le dédoublement de la (±)(diméthylamino-3 méthyl-2 propyl)-10 méthoxy-2 phénothiazine par l'acide (-) L-di.p.toluyl-tartrique s'effectue selon les quatre étapes suivantes :

1) cristallisation du sel neutre d'acide di.p.toluyl-tartrique et de la base dextrogyre,
2) cristallisation du maléate acide de la base lévogyre (maléate acide de lévomépromazine),
3) isolement de lbase racémique restante,
4) récupération de l'acide di.p.toluyl-tartrique en vue de recyclage.

Généralement, le sel neutre d'acide di.p.toluyl-tartrique avec la base dextrogyre est obtenu en refroidissant progressivement une solution chaude (environ 60°C) et agitée d'un mélange de (±)(diméthylamino-3 méthyl-2 propyl)-10 méthoxy-2 phénothiazine et d'acide (-) L-di.p.toluyl-tartrique dans un solvant organique tel que l'éthanol, dans lequel le sel neutre de la base dextrogyre est peu soluble ou insoluble à une température voisine de 20°C, jusqu'à une température voisine de 20°C, après amorçage de la cristallisation par quelques cristaux du sel neutre de l'acide di.p.toluyl-tartrique avec la base dextrogyre. Généralement la cristallisation du sel neutre de l'acide di.p.toluyl-tartrique avec la base dextrogyre est complète au bout de 4 heures.

De préférence, on utilise 0,25 à 0,5 mole d'acide (–) L-di.p.toluyl-tartrique par mole de base racémique mise en oeuvre, éventuellement en présence d'acide formique.

La quantité de solvant est celle qui est suffisante pour dissoudre à chaud le mélange de base racémique et d'agent de dédoublement.

Généralement, le maléate acide de lévomépromazine est obtenu en ajoutant au filtrat provenant de la séparation et du lavage du sel neutre de la base dextrogyre une quantité suffisante d'acide maléique en solution dans le même solvant. De préférence, on utilise une mole d'acide maléique par mole de base racémique dédoublée. Le maléate acide de lévomépromazine qui précipite est séparé par filtration.

Selon le procédé de la présente invention, la base racémique qui n'a pas été dédoublée peut être récupérée après concentration puis alcalinisation, par exemple au moyen d'une solution aqueuse de carbonate de sodium, des eaux-mères de cristallisation du maléate acide de lévomépromazine et enfin extraction par un solvant convenable.

Par ailleurs, l'acide (–) L-di.p.toluyl-tartrique utilisé comme agent de dédoublement, qui est présent dans le sel neutre de la base dextrogyre et dans les eaux de lavage alcalines provenant del'isolement de la base racémique non dédoublée, peut être récupéré et recyclé.

Par exemple, la base dextrogyre est déplacée de son sel par action d'une base et l'acide (-) L-di.p.toluyl-tartrique est obtenu après acidification de ses solutions basiques. Généralement, il est possible de récupérer plus de 80 % de l'acide (-) L-di.p.toluyl-tartrique mis en oeuvre.

Le procédé selon la présente invention permet de préparer le maléate acide de lévomépromazine avec des rendements généralement supérieurs à 85 % à partir de la base racémique et en utilisant une quantité faible d'agent de dédoublement.

Les exemples suivants, donnés à titre non limitatif, montrent comment l'invention peut être mise en pratique.

### EXEMPLE 1

a) Dans un ballon tricol de 250 cm3 muni d'un agitateur mécanique, d'un réfrigérant et d'une arrivée d'azote, on introduit 16,5 g (0,05 mole) de (±)(diméthylamino-3 méthyl-2 propyl)-10 méthoxy-2 phénothiazine, 10,15 g (0,025 mole) d'acide (-) L-di.p.toluyl-tartrique monohydraté et 75 cm3 d'éthanol. Le mélange agité est chauffé à 60°C jusqu'à obtention d'une solution homogène. On refroidit à 50°C puis on amorce la cristallisation en ajoutant quelques cristaux de sel neutre d'acide di.p.toluyl-tartrique et de base dextrogyre. Tout en agitant, on maintient pendant 1 heure à 50°C, refroidit à 20°C en 2 heures et maintient à 20°C pendant 1 heure. Les cristaux obtenus sont séparés par filtration, lavés deux fois par 30 cm3 d'éthanol puis séchés. On obtient ainsi 12,99 g de sel neutre d'acide di.p.toluyl-tartrique et de (diméthylamino-3 méthyl-2 propyl)-10 méthoxy-2 phénothiazine dextrogyre.

b) Au filtrat et aux lavages de cristallisation du sel neutre obtenu ci-dessus, on ajoute une solution de 2,94 g (0,025 mole) d'acide maléique dans 11 cm3 d'éthanol. Des cristaux se développent spontanément. On maintient pendant 2 heures sous agitation à une température voisine de 20°C puis pendant 18 heures à +4°C.

Après filtration on obtient, avec un rendement de 88 %, 9,76 g de maléate acide de lévomépromazine dont le pouvoir rotatoire est :

$[\alpha]_D^{20}$ = -7,7° (c = 5, diméthylformamide)

La base libérée à partir d'une partie aliquote a un pouvoir rotatoire de :

$[\alpha]_D^{20}$ = 15,15° (c = 5, chloroforme).

c) Les eaux-mères de cristallisation du maléate acide de lévomépromazine sont concentrées à sec sous pression réduite. Le résidu est repris par 50 cm3 de chlorure de méthylène puis on ajoute une solution de 6 g de carbonate de sodium dans 60 cm3 d'eau. Le mélange est agité pendant 1 heure. La phase aqueuse qui est séparée par décantation est lavée par 10 cm3 de chlorure de méthylène. La phase organique est lavée par 50 cm3 d'eau puis séchée sur sulfate de sodium. Après filtration et concentration sous pression réduite, on obtient 1,64 g de (±)(diméthylamino-3 méthyl-2 propyl)-10 méthoxy-2 phénothiazine.

d) On dissout 12,99 g de sel neutre d'acide di.p.toluyl-tartrique et de (diméthylamino-3 méthyl-2 propyl)-10 méthoxy-2 phénothiazine dans 50 cm3 de chlorure de méthylène. On ajoute 70 cm3 d'eau et ajuste le pH à 11 par addition de 27,3 cm3 de soude N. La phase chlorométhylénique est séparée par décantation et séchée sur sulfate de sodium. Après filtration et concentration sous pression réduite, on obtient 7,53 g de (+)(diméthylamino-3 méthyl-2 propyl)-10 méthoxy-2 phénothiazine dont le pouvoir rotatoire est :

$[\alpha]_D^{20}$ = +15,8° (c = 5, chloroforme)

La phase aqueuse (pH = 11) et les eaux de lavage provenant de l'isolement de la base racémique sont réunies. On ajoute 100 cm3 d'acétate d'éthyle et ajuste le pH à 2 par addition de 69,3 cm3 d'acide chlorhydrique 2N. La phase organique est séparée par décantation puis séchée sur sulfate de sodium. Après filtration et concentration à sec sous pression réduite, on obtient 8,75 g (rendement : 86 %) d'acide (-) L-di.p.toluyl-tartrique dont le pouvoir rotatoire est :

$[\alpha]_D^{20}$ = -122,8° (c = 1, éthanol).

EXEMPLE 2

a) Dans un ballon tricol de 100 cm3 muni d'un agitateur mécanique, d'un réfrigérant et d'une arrivée d'azote, on introduit 6,57 g (0,02 mole) de (±)(diméthylamino-3 méthyl-2 propyl)-10 méthoxy-2 phénothiazine, 2,09 g d'acide (-) L-di.p.toluyl-tartrique monohydraté, 0,38 cm3 d'acide formique à 98 % et 30 cm3 d'éthanol. Le mélange agité est chauffé à 60°C jusqu'à obtention d'un milieu homogène. On refroidit à 50°C puis on ajoute quelques cristaux de sel neutre d'acide di.p.toluyl-tartrique de base dextrogyre.

On maintient la température pendant 1 heure à 50°C puis refroidit de 50°C à 20°C en 2 heures. On maintient pendant 1 heure à 20°C sous agitation. Les cristaux obtenus sont séparés par filtration, lavés 2 fois par 12 cm3 d'éthanol et séchés. On obtient ainsi 3,98 g de sel neutre d'acide di.p.toluyl-tartrique et de (diméthylamino-3 méthyl-2 propyl)-10 méthoxy-2 phénothiazine dextrogyre dont le pouvoir rotatoire est :

$[\alpha]_D^{20}$ = +16.6° (c = 5, chloroforme).

b) Au filtrat et aux lavages de cristallisation du sel neutre obtenu ci-dessus, on ajoute 1,16 g d'acide maléique. Des cristaux se développent spontanément à une température voisine de 20°C. On agite pendant 2 heures à une température voisine de 20°C puis pendant 18 heures à 4°C. Après filtration, on obtient 3,52 g de maléate acide de lévomépromazine.

La base libérée à partir d'une partie aliquote a un pouvoir rotatoire de :

$[\alpha]_D^{20}$ = -13,7° (c = 5, chloroforme).

## Revendications

1. Procédé de préparation du maléate acide de lévomépromazine à partir de la (±)(diméthylamino-3 méthyl-2 propyl)-10 méthoxy-2 phénothiazine caractérisé en ce que l'on dédouble la (±)(diméthylamino-3 méthyl-2 propyl)-10 méthoxy-2 phénothiazine au moyen d'acide (-) L-di.p.toluyl-tartrique pour obtenir le sel neutre de l'acide di.p.toluyl-tartrique et de la (diméthylamino-3 méthyl-2 propyl)-10 méthoxy-2 phénothiazine puis précipite le maléate acide de lévomépromazine par action de l'acide maléique sur les eaux-mères de cristallisation du sel neutre de la base dextrogyre et, éventuellement isole la base racémique non dédoublée et régénère l'acide (-) L-di.p.toluyl-tartrique.

2. Procédé selon la revendication 1 caractérisé en ce que l'on opère dans l'éthanol utilisé en quantité suffisante pour dissoudre à chaud le mélange de (±)(diméthylamino-3 méthyl-2 propyl)-10 méthoxy-2 phénothiazine et l'acide (-) L-di.p.toluyl-tartrique.

3. Procédé selon la revendication 1 caractérisé en ce que l'on utilise 0,25 à 0,5 mole d'acide (-) L-di.p.toluyl-tartrique par mole de (±)(diméthylamino-3 méthyl-2 propyl)-10 méthoxy-2 phénothiazine.

4. Procédé selon la revendication 1 caractérisé en ce que l'on utilise une mole d'acide maléique par mole de base racémique dédoublée.

## Patentansprüche

1. Verfahren zur Herstellung von saurem Levomepromazinmaleat aus (±)-10-(3-Dimethylamino-2-methylpropyl)-2-methoxy-phenothiazin, dadurch gekennzeichnet, daß man (±)-10-(3-Dimethylamino-2-methylpropyl)-2-methoxy-phenothiazin mit Hilfe von (-)-L-Di-p-toluylweinsäure unter Bildung des neutralen Salzes von Di-p-toluyl-weinsäure und 10-(3-Dimethylamino-2-methylpropyl)-2-methoxy-phenothiazin spaltet, dann das saure Levomepromazinmaleat durch Einwirkung von Maleinsäure auf die Kristallisationsflüssigkeit des neutralen Salzes der rechtsdrehenden Base fällt, gegebenenfalls die nicht gespaltene racemische Base isoliert und die (-)-L-Di-p-toluylweinsäure regeneriert.

2. Verfahren nach Anspruch 1, dadurch gekennzeichnet, daß man in Äthanol arbeitet, das in zur Heißlösung der Mischung von (±)-10-(3-Dimethyl-amino-2-methylpropyl)-2-methoxy-phenothiazin und (-)-L-Di-p-toluylweinsäure ausreichender Menge verwendet wird.

3. Verfahren nach Anspruch 1, dadurch gekennzeichnet, daß man 0,25 bis 0,5 Mol (-)-L-Di-p-toluyl-weinsäure pro mol (±)-10-(3-Dimethylamino-2-methylpropyl)-2-methoxy-phenothiazin einsetzt.

4. Verfahren nach Anspruch 1, dadurch gekennzeichnet, daß man ein Mol Maleinsäure pro Mol gespaltener racemischer Base verwendet.

**Claims**

1. A process for preparing levomepromazine hydrogen maleate from (±)-10-(3-dimethylamino-2-methylpropyl)-2-methoxyphenothiazine, wherein (±)-10-(3-dimethylamino-2-methylpropyl)-2-methoxyphenothiazine is resolved by means of (-)-di(p-toluoyl)-L-tartaric acid to obtain the neutral salt of di(p-toluoyl)tartaric acid and 10-(3-dimethylamino-2-methylpropyl)-2-methoxyphenothiazine, the levomepromazine hydrogen maleate is then precipitated by the action of maleic acid on the mother liquors of crystallization of the neutral salt of the dextrorotatory base and, optionally, the unresolved racemic base is isolated and the (-)-di(p-toluoyl)-L-tartaric acid regenerated.

2. The process according to claim 1, which is carried out in ethanol used in a sufficient amount to dissolve the mixture of (±)-10-(3-dimethylamino-2-methylpropyl)-2-methoxyphenothiazine and (-)-di(p-toluoyl)-L-tartaric acid in the hot state.

3. The process according to claim 1, wherein 0.25 to 0.5 mole of (-)-di(p-toluoyl)-L-tartaric acid is used per mole of (±)-10-(3-dimethylaeino-2-methylpropyl)-2-methoxyphenothiazine.

4. The process according to claim 1, wherein one mole of maleic acid is used per mole of racemic base resolved.